**Europäisches Patentamt**

**European Patent Office**

**Office européen des brevets**

(19)

(11) Numéro de publication: **0 014 687**
**B1**

(12) # FASCICULE DE BREVET EUROPEEN

(45) Date de publication du fascicule du brevet:
**16.03.83**

(21) Numéro de dépôt: **80810039.0**

(22) Date de dépôt: **04.02.80**

(51) Int. Cl.³: **A 61 B 5/10,** G 01 L 5/03,
A 63 C 9/08, G 01 R 29/22

(54) **Procédé pour déterminer des contraintes par mesure et analyse de potentiels piézo-électriques produits dans un matériau organique rigide, dispositif pour la mise en oeuvre de ce procédé et application de ce procédé.**

(30) Priorité: **05.02.79 CH 1085/79**

(43) Date de publication de la demande:
**20.08.80 Bulletin 80/17**

(45) Mention de la délivrance du brevet:
**16.03.83 Bulletin 83/11**

(84) Etats contractants désignés:
**AT CH DE FR IT SE**

(56) Documents cités:
**FR-A-2 100 323**
**FR-A-2 198 762**
**FR-A-2 266 527**
**FR-A-2 269 981**
**FR-A-2 351 678**
**FR-A-2 418 655**
**US-A-3 826 509**

**IEEE TRANSACTIONS ON BIOMEDICAL ENGINEER-ING, vol. BME-24, no. 6, novembre 1977 New York US S. SARA et R.S. LAKES: «A Non-Invasive Technique for Detecting Stress Waves in Bone Using the Piezo-electric Effect», pages 508–512**

(73) Titulaire: **BATTELLE MEMORIAL INSTITUTE, 7 route de Drize, CH-1227 Carouge/Genève (CH)**

(72) Inventeur: **Arieh, Simon, 24, Av. de la Gare des Eaux-Vives, CH-1208 Geneve (CH)**
Inventeur: **Courvoisier, Guy, 38, rue du Grand-Pré, CH-1202 Geneve (CH)**
Inventeur: **Prost, Jean-Louis, 3, rue de l'Université, CH-1205 Geneve (CH)**

(74) Mandataire: **Dousse, Blasco et al, 7, route de Drize, CH-1227 Carouge/Genève (CH)**

## Procédé pour déterminer des contraintes par mesure et analyse de potentiels piézo-électriques produits dans un matériau organique rigide, dispositif pour la mise en œuvre de ce procédé et application de ce procédé

L'invention se rapporte à un procédé pour déterminer des contraintes par mesure et analyse de potentiels d'origine piézo-électrique produits dans un matériau organique rigide en forme d'organe allongé assimilable mécaniquement à une poutre encastrée à une extrémité, consécutivement à une sollicitation mécanique exercée sur cet organe.

Des travaux scientifiques ont montré que différents corps organiques produisent des variations de potentiels électriques lorsqu'ils se déforment. C'est notamment le cas des muscles ou des os.

C'est ainsi que l'on a déjà effectué de nombreux travaux pour étudier la vitesse de transmission ou l'atténuation d'amplitude d'ondes ultrasoniques utilisées pour exciter un os. Des détecteurs sont placés le long de l'os, de part et d'autre de la fracture, et les signaux détectés sont comparés.

Il a, par ailleurs, été proposé dans la demande de brevet FR 2 198 762, de commander l'ouverture d'une fixation de ski de sécurité en utilisant des capteurs piézo-électriques pour mesurer séparément des contraintes de flexion et de torsion s'exerçant sur cette fixation, les signaux détectés étant ensuite amplifiés et intégrés. Dans un tel procédé, la mesure des contraintes est indirecte puisqu'elle est mesurée sur un élément de la fixation et ne correspond donc pas forcément aux contraintes appliquées au tibia.

Il a également été proposé d'utiliser les signaux d'origine musculaire pour commander l'ouverture automatique d'une fixation de ski en cas de risque de fracture de la jambe du skieur. Un tel dispositif de sécurité fait l'objet des brevets DE 2 121 827, US 3 776 566 et 3 826 509.

Cette solution pose cependant plus de problème qu'elle n'en résout. En effet, si l'on considère que toute activité musculaire est génératrice d'une variation de potentiel électrique, ces brevets restent muets sur la manière d'utiliser ces courants à bon escient pour générer un signal de commande de l'ouverture de la fixation. La pratique du ski s'accompagne obligatoirement d'un travail musculaire. Or l'ouverture de la fixation ne doit intervenir qu'exceptionnellement au moment où les sollicitations des os de la jambe deviennent anormales. Il est, par conséquent, nécessaire de définir un seuil à partir duquel on désire générer un signal issu du potentiel électrique d'origine musculaire. La définition de ce seuil pose des problèmes extrêmement complexes, étant donné que les sollicitations auxquelles l'os est soumis et celles qui s'exercent sur les muscles ne sont pas forcément proportionnelles ou simultanées. En outre, les potentiels électriques d'origine musculaire ne permettent pas de déterminer le type de contrainte auquel les os sont soumis; or la résistance d'un os n'est pas la même s'il est soumis à une flexion ou à une torsion. Ceci signifie que les potentiels électriques d'origine musculaire peuvent, dans certains cas, dépasser le seuil limite, alors que la sollicitation de l'os est parfaitement tolérable, tandis que dans d'autres cas, ce seuil limite n'est pas atteint quand l'os est soumis à une contrainte dépassant sa limite de résistance.

Les défauts et les dangers de la solution proposée apparaissent immédiatement et semblent inhérents à la conception même de ce dispositif de sécurité, étant donné qu'il mesure des potentiels électriques dépendant de l'activité musculaire et que ces potentiels sont censés être caractéristiques des sollicitations exercées sur les os.

Des travaux scientifiques ont montré, par ailleurs, que des effets électriques se manifestent lorsque des organes rigides en matériaux organiques tels que le bois ou les os sont soumis à des sollicitations mécaniques. Ces essais ont permis de constater que les formes de signaux engendrés par les potentiels électriques consécutifs aux sollicitations exercées sur les os sont différentes suivant la nature de la contrainte et que l'amplitude du signal est fonction à la fois de l'importance de la contrainte et de sa vitesse d'application. Cela signifie que, pour un niveau de contrainte donné, le signal mesuré va dépendre de la vitesse d'application de la contrainte.

En tenant compte de ces données, il apparaît qu'il doit être possible de mesurer le type et l'amplitude des contraintes exercées sur un os ou sur une tige de bois, de manière à comparer le résultat avec une contrainte type. Cette comparaison peut être utilisée à différentes fins, notamment pour déclencher une fixation de ski lorsque la contrainte relevée sur la jambe du skieur dépasse une limite admissible ou à d'autres fins que l'on énumérera par la suite.

A cet effet, la présente invention a pour objet un procédé pour déterminer des contraintes par mesure et analyse de potentiels piézo-électriques produits dans un matériau organique rigide en forme d'organe allongé assimilable mécaniquement à une poutre encastrée à une extrémité, consécutivement à des sollicitations mécaniques exercées sur cet organe, caractérisé par le fait que l'on détecte ces potentiels en deux points dudit organe situés à une distance déterminée l'un de l'autre, on amplifie et intègre les signaux détectés en chacun de ces points, on détermine la valeur de la contrainte de flexion s'exerçant au point de détection le plus éloigné du point d'encastrement en effectuant la différence entre ces signaux et en multipliant le résultat par le rapport des distances entre ce point de détection le plus éloigné du point d'encastrement et d'une part, ce point d'encastrement, d'autre part, l'autre des points de détection et on détermine la valeur de la contrainte de torsion en effectuant la différence entre le plus grand des signaux intégrés et ladite contrainte de flexion.

Cette invention a encore pour objet un dispositif pour la mise en œuvre de ce procédé, caractérisé par le fait qu'il comporte deux électrodes destinées à être disposées le long dudit organe rigide et reliées chacune à un circuit intégrateur, un premier opérateur analogique pour établir la différence entre les signaux issus des circuits intégrateurs, un second opérateur analogique pour multiplier la valeur issue du premier opérateur analogique par le rapport entre la distance au point d'encastrement dudit organe rigide de l'électrode la plus éloignée de ce point et la distance entre ces électrodes pour faire apparaître à sa sortie la variation de la contrainte de flexion, et un troisième opérateur analogique dont les entrées sont reliées d'une part, à la sortie du second opérateur et, d'autre part à la sortie du circuit intégrateur lié à l'électrode la plus éloignée du point d'encastrement pour faire apparaître à sa sortie la valeur de la contrainte de torsion.

Le dessin annexé illustre, schématiquement et à titre d'exemple, un mode de mise en œuvre du procédé objet de l'invention.

La fig. 1 illustre un schéma électronique de traitement des signaux captés.

Les figs. 2 et 3 sont des diagrammes explicatifs.

Meilleure manière de réaliser l'invention

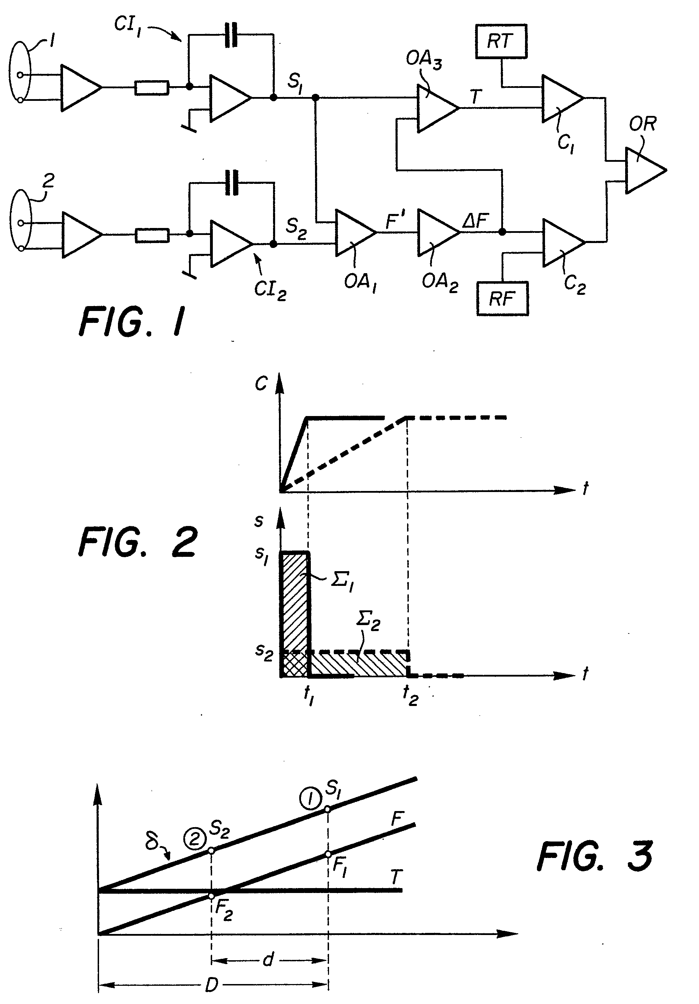

Dans le cas où la détection des potentiels piézoélectriques provenant des os est destinée à déclencher une fixation de ski par exemple dès qu'un seuil de contrainte prédéterminé a été atteint, il est nécessaire de mesurer indépendamment les deux composantes dont est formé le signal afin de savoir quelle est la répartition entre contraintes de torsion et contraintes de flexion. Il est bien connu de la théorie sur la résistance des matériaux que, dans le cas d'une torsion, la contrainte est constante en tous les points d'une poutre encastrée à une extrémité, tandis qu'avec une flexion, la contrainte croît en direction du point d'encastrement. Il est d'autre part également connu que la tension d'origine piézo-électrique est dépendante de la vitesse d'application de la contrainte. Comme le montre le diagramme de la fig. 2, l'application d'une contrainte C pendant un temps $t_1$ donne, pendant le même temps $t_1$, un signal $s_1$, tandis que l'application de la même contrainte C pendant un temps $t_2$ plus long donne un signal $s_2$ d'amplitude plus faible pendant le même temps $t_2$. Par contre, les surfaces $\varepsilon_1$ et $\varepsilon_2$ sont égales; par conséquent, en intégrant le signal S, on introduit la notion du paramètre «temps» et cette intégration nous ramène à la contrainte elle-même plutôt qu'à sa dérivée.

Ce résultat peut être obtenu à l'aide de deux circuits intégrateurs actifs à amplificateurs opérationnels illustrés par la fig. 1 qui comprend essentiellement deux électrodes de mesure 1 et 2, chacune associée à un circuit intégrateur actif à amplificateur opérationnel $CI_1$ et $CI_2$ à la sortie desquels apparaissent des signaux $S_1$, respectivement $S_2$, caractéristiques des potentiels et du temps mesurés. Ensuite, ces signaux sont traités par une série d'opérateurs analogiques agencés pour effectuer le développement mathématique que l'on va exposer en se référant au diagramme de la fig. 3.

Ce diagramme montre une droite δ qui correspond à la somme de deux contraintes respectivement de torsion T et de flexion F d'une poutre encastrée, la partie inférieure de la jambe, notamment le tibia, pouvant être assimilée à ce cas. Les signaux $S_1$ et $S_2$ mesurés en deux points 1 et 2 de cette droite δ, écartés d'une distance d, $S_1$ étant à une distance D du point d'encastrement, correspondent à la somme des deux contraintes de torsion et de flexion en ces points de la droite δ.

$$S_1 = T + F_1$$

$$S_2 = T + F_2$$

$$S_1 - S_2 = F_1 - F_2 = F'$$

La droite δ a une équation de la forme:

$$y = ax + b$$

a) est la pente de $\delta = \dfrac{F'}{d}$

b) est l'ordonnée à l'origine = T au point I de la droite δ

$$S_1 = \frac{F'}{d} D + T$$

ou encore $S_1 = \dfrac{D}{d} F' + T$

Dans cette équation, T est l'inconnue

$$T = S_1 \frac{D}{d} F' \qquad = S_1 - \Delta F'$$

$$F_1 = \frac{D}{d} F' \qquad = \Delta F'$$

Pour résoudre cette équation à partir des signaux $S_1$ et $S_2$ issus des circuits intégrateurs actifs $CI_1$ et $CI_2$, un opérateur analogique $OA_1$ dont les deux entrées sont reliées aux sorties de $CI_1$ et $CI_2$ établit la différence entre ces signaux $S_1$ et $S_2$, de sorte que la valeur F' apparaît à la sortie de cet opérateur $OA_1$. Un second opérateur analogique $OA_2$ multiplie cette valeur F' par Δ qui correspond à $\dfrac{D}{d}$ La valeur Δ F sortant de l'opérateur $OA_2$ est dirigée d'une part vers une entrée d'un opérateur analogique $OA_3$ dont la seconde entrée est reliée à la sortie de l'intégrateur $CI_1$. Cet opérateur $OA_3$ établit la différence entre $S_1$ et Δ F, de sorte que la valeur T apparaît à sa sortie.

Deux comparateurs $C_1$ et $C_2$ comparent respectivement les valeurs de T et de Δ F' avec une valeur de référence RT et RF correspondant, par exemple, dans le cas d'une fixation de ski de sécurité, à la limite admissible respectivement de torsion et de flexion. Ces deux comparateurs qui émettent un signal dès que l'une de ces limites est dépassée sont reliés à une porte «OR» destinée à fournir le signal utile commandant le déclenchement de la fixation.

Bien entendu, la qualité et la précision des mesures effectuées à l'aide du procédé décrit dépendent essentiellement de la propreté du signal capté que l'on amplifie. Cette propreté peut être influencée par divers facteurs, notamment par des potentiels ne provenant pas de l'os, mais par exemple de l'activité musculaire qui accompagne forcément les contraintes appliquées sur l'os lors de la pratique du ski notamment.

Des essais de mesure de ces potentiels in vivo ont été menés aussi bien en laboratoire que sur la jambe de skieurs évoluant sur le terrain. Ces essais ont été réalisés tout d'abord avec une paire d'électrodes simples. Les potentiels relevés à l'aide de ces électrodes ont bien permis de mesurer des variations en fonction des contraintes appliquées à l'os; toutefois les diagrammes relevés à l'aide d'un enregistreur utilisé pour les électrocardiogrammes ont révélé une proportion importante de potentiels parasites, de sorte que le traitement d'un signal issu de cette électrode est difficilement exploitable, étant donné que, pour certaines contraintes, l'amplitude du signal provenant des variations de potentiel engendrées par l'effet piézo-électrique dû à l'os peut être du même ordre de grandeur que les potentiels parasites.

Ensuite, des essais ont été réalisés avec une paire de doubles électrodes, l'électrode de mesure étant entourée d'un anneau de garde. Pour améliorer le contact entre l'électrode et la peau, on a utilisé une crème conductrice du type de celles utilisées pour l'enregistrement des électrocardiogrammes. Les électrodes ont été placées le long du tibia qui est directement adjacent à la peau sur une portion importante de sa longueur.

La propreté du signal enregistré dans ces conditions est nettement meilleure que précédemment et permet d'envisager un traitement du signal selon le procédé décrit précédemment.

Possibilité d'exploitation industrielle

Bien entendu, le procédé objet de l'invention n'est pas seulement utilisable dans le cas où l'on désire produire un signal de déclenchement d'une fixation de ski. Cette application n'est donnée qu'à titre d'exemple. On peut, en effet, envisager d'autres applications, par exemple médicales, en vue de détecter une fracture ou une fissure ou l'état de guérison d'une fracture. On peut aussi envisager des applications dans le domaine du sport, notamment de l'entraînement sportif; on peut en effet imaginer de détecter les contraintes du tibia d'un athlète qui saute ou qui court. Le tibia n'est pas le seul os dont on peut mesurer les potentiels d'origine piézo-électrique, les os de l'avant-bras sont également proches de la peau, de sorte que l'on peut, dans ce cas, envisager par exemple de mesurer les contraintes résultant de la pratique du tennis et de là, relever des défauts dans la technique d'un joueur. Ce procédé n'est évidemment pas limité aux os, mais est applicable à d'autres organes rigides en matériau organique tel que le bois, de sorte qu'il est possible de mesurer l'état des contraintes s'exerçant sur des structures en bois.

**Revendications**

1. Procédé pour déterminer des contraintes par mesure et analyse de potentiels piézo-électriques produits dans un matériau organique rigide en forme d'organe allongé assimilable mécaniquement à une poutre encastrée à une extrémité, consécutivement à des sollicitations mécaniques exercées sur cet organe, caractérisé par le fait que l'on détecte ces potentiels en deux points (1, 2) dudit organe situés à une distance (d) déterminée l'un de l'autre, on amplifie et intègre les signaux détectés ($S_1$, $S_2$) en chacun de ces points, on détermine la valeur de la contrainte de flexion (F) s'exerçant au point de détection le plus éloigné du point d'encastrement en effectuant la différence ($S_1 - S_2 = F'$) entre ces signaux et en en multipliant le résultat (F') par le rapport (Δ) des distances (D, d) entre ce point de détection (1) le plus éloigné du point d'encastrement et d'une part, ce point d'encastrement, d'autre part l'autre (2) des points de détection et on détermine la valeur de la contrainte de torsion (T) en en effectuant la différence entre le plus grand ($S_1$) des signaux intégrés et ladite contrainte de flexion (Δ F').

2. Procédé selon la revendication 1, caractérisé par le fait que l'on compare chacune des contraintes mesurées à une valeur de référence.

3. Procédé selon la revendication 1, caractérisé par le fait que l'on génère un signal de commande chaque fois qu'au moins une des valeurs mesurées dépasse la valeur de référence.

4. Dispositif pour la mise en œuvre du procédé selon la revendication 1, caractérisé par le fait qu'il comporte deux électrodes (1, 2) destinées à être disposées le long dudit organe rigide et reliées chacune à un circuit intégrateur ($CI_1$, $CI_2$), un premier opérateur analogique ($OA_1$) pour établir la différence entre les signaux ($S_1$, $S_2$) issus des circuits intégrateurs ($CI_1$, $CI_2$), un second opérateur analogique ($OA_2$) pour multiplier la valeur (F') issue du premier opérateur analogique ($OA_1$) par le rapport (Δ) entre la distance (D) au point d'encastrement dudit organe rigide de l'électrode la plus éloignée de ce point et la distance (d) entre ces électrodes (1, 2) pour faire apparaître à sa sortie la variation (ΔF') de la contrainte de flexion, et un troisième opérateur analogique ($OA_3$) dont les entrées sont reliées d'une part, à la sortie du second opérateur analogique ($OA_2$) et, d'autre part, à la sortie du circuit intégrateur $CI_1$ lié à l'électrode (1) la plus éloignée du point d'encastrement pour faire apparaître à sa sortie la valeur de la contrainte de torsion.

5. Dispositif selon la revendication 4, caractérisé par le fait qu'il comporte en plus deux comparateurs ($C_1$, $C_2$) dont les entrées respectives sont reliées aux sorties des deuxième et troisième opérateurs analogiques ($OA_2$, $OA_3$) ainsi qu'à des valeurs de référence respectivement de torsion et de flexion (RT, RF).

6. Application du procédé selon la revendication 1 pour l'ouverture d'une fixation de ski de sécurité, dès que l'une desdites contraintes mesurées dépasse un seuil déterminé, les signaux $S_1$ et $S_2$ étant mesurés sur la jambe du skieur.

## Patentansprüche

1. Verfahren zum Bestimmen von Belastungen durch Messen und Analysieren piezo-elektrischer Potentiale, die in einem starren organischen Material in Form eines langgestreckten Körpers, mechanisch vergleichbar mit einem an einem Ende eingespannten Balken, auftreten, aufgrund von mechanischen, auf diesen Körper ausgeübten Beanspruchungen, dadurch gekennzeichnet, dass man diese Potentiale an zwei Punkten (1, 2) des Körpers feststellt, die voneinander in einem festgelegten Abstand (d) angeordnet sind, dass man die an jedem dieser Punkte festgestellten Signale ($S_1$, $S_2$) verstärkt und integriert, dass man den Wert (F) der Biegebeanspruchung bestimmt, der am vom Einspannpunkt am weitesten entfernten Messpunkt auftritt, indem die Differenz ($S_1$–$S_2$ = F') zwischen diesen Signalen gebildet wird und indem man das Ergebnis (F') mit dem Verhältnis (Δ) der Abstände (D,d) zwischen dem am weitesten vom Einspannpunkt entfernten Messpunkt (1) und diesem Einspannpunkt einerseits sowie dem anderen Messpunkt (2) anderseits multipliziert, und dass man den Wert der Torsionsbeanspruchung (T) bestimmt, indem man die Differenz zwischen dem grössten integrierten Signal ($S_1$) und der Biegebeanspruchung (Δ, F') bildet.

2. Verfahren nach Anspruch 1, dadurch gekennzeichnet, dass man jede der gemessenen Beanspruchungen mit einem Bezugswert vergleicht.

3. Verfahren nach Anspruch 1, dadurch gekennzeichnet, dass man jedes Mal, wenn wenigstens einer der gemessenen Werte den Bezugswert überschreitet, ein Steuersignal erzeugt.

4. Anordnung zur Durchführung des Verfahrens nach Anspruch 1, dadurch gekennzeichnet, dass sie zwei Elektroden (1, 2), die entlang des starren Körpers angeordnet werden können und jeweils mit einem Integrationskreis ($CI_1$, $CI_2$) verbunden sind, einen ersten Analogoperator ($OA_1$), um die Differenz zwischen den Signalen ($S_1$, $S_2$) zu bilden, die von den Integrationskreisen ($CI_1$, $CI_2$) austreten, einen zweiten Analogoperator ($OA_2$), um den vom ersten Analogoperator ($OA_1$) abgegebenen Wert (F') mit dem Verhältnis (Δ) zwischen dem Abstand (D) des Einspannpunktes des starren Körpers von der von diesem Punkt am weitesten entfernten Elektrode und dem Abstand (d) zwischen diesen Elektroden (1, 2) zu multiplizieren, um an seinem Ausgang die Veränderung (Δ,F') der Biegebelastung aufscheinen zu lassen, und einen dritten Analogoperator ($OA_3$) aufweist, dessen Eingänge einerseits mit dem Ausgang des zweiten Analogoperators ($OA_2$) und anderseits mit dem Ausgang des Integrationskreises ($CI_1$) verbunden sind, der mit der vom Einspannpunkt am weitesten entfernten Elektrode (1) verbunden ist, um an seinem Ausgang den Wert der Torsionsbelastung aufscheinen zu lassen.

5. Anordnung nach Anspruch 4, dadurch gekennzeichnet, dass sie zusätzlich zwei Komparatoren ($C_1$, $C_2$) umfasst, deren jeweilige Eingänge mit den Ausgängen des zweiten bzw. dritten Analogoperators ($OA_2$, $OA_3$) sowie mit den Bezugswerten für die Torsion bzw. für die Biegung (RT, RF) verbunden sind.

6. Anwendung des Verfahrens nach Anspruch 1 für das Öffnen einer Sicherheitsskibindung, sobald eine der gemessenen Belastungen eine festgelegte Schwelle überschreitet, wobei die Signale ($S_1$, $S_2$) am Bein des Skiläufers gemessen werden.

## Claims

1. A process for the determination of strengths in an elongated rigid beam-like organic material element fixedly supported at one end by measuring and analyzing the piezo-electric potentials produced therein consecutive to the application of mechanical forces to said element, characterized in that one detects the potentials present at a first and a second point (1, 2) of said element, said points being separated from each other by a known distance (d), one amplifies and integrates the detected signals ($S_1$, $S_2$) at each point, one determines the bending strength applied to that point (1) of detection which is the most distant from said supporting end by substracting one of said signals from the other ($S_1$–$S_2$ = F') and multiplying the result (F') by the ratio (Δ) of the distance (D/d) between the first point (1) of detection and said supporting end and between the two points and one determines the torsional strength by substracting said bending strength value (ΔF') from the larger ($S_1$) of said detected signals.

2. Process according to claim 1, characterized in that one compares each measured strength with a reference value.

3. Process according to claim 1, characterized in that one provides a driving signal each time when at least one of the measured value exceeds the reference value.

4. Device for embodying the process according to claim 1, characterized in comprising two electrodes (1, 2) to be disposed against said rigid element and connected each to an integrator circuit ($CI_1$, $CI_2$), a first analog operator ($OA_1$) for establishing the difference between the signals ($S_1$, $S_2$) from the integrator circuits ($CI_1$, $CI_2$), a second analog operator ($OA_2$) for multiplying the value (F') from the first analog operator ($OA_1$) by the ratio (Δ) of the distance (D) of the most distant electrode from the supporting end of said rigid element over the distance (d) between the electrodes (1, 2) for providing at the output the variation (ΔF') of the bending strength, and a third analog operator ($OA_3$) whose inputs are connected to the output of the second analog operator ($OA_2$) on one hand, and to the output of the integrator circuit ($C_1$) connected to the electrode the most distant from the supporting end on the other hand, for providing at the output thereof the torsional strength value.

5. Device according to claim 4, characterized in comprising, in addition, two comparators ($C_1$, $C_2$) whose respective inputs are connected to the outputs of the second and third analog operators ($OA_2$, $OA_3$) and also to sources of reference values of torsional strength and flexural strength (RT, RF), respectively.

6. Application of the process according to claim 1, for controlling the opening of a safety ski binder when one of the measured strength value exceeds a predetermined level, the signals $S_1$ and $S_2$ being measured on the leg of the skier.

FIG. 1

FIG. 2

FIG. 3